(19) Europäisches Patentamt
European Patent Office
Office européen des brevets

(11) Publication number: 0 552 665 A1

(12) # EUROPEAN PATENT APPLICATION

(21) Application number: 93100451.9

(51) Int. Cl.$^5$: C07D 403/04, A61K 31/55

(22) Date of filing: 14.01.93

(30) Priority: 24.01.92 US 825475

(43) Date of publication of application: 28.07.93 Bulletin 93/30

(84) Designated Contracting States: AT BE CH DE DK ES FR GB GR IE IT LI LU MC NL PT SE

(71) Applicant: F. HOFFMANN-LA ROCHE AG
Postfach 3255
CH-4002 Basel(CH)

(72) Inventor: Hsu, Ming-Chu
445 East 86th Street, Apt. 15G
New York, N.Y. 10028(US)

(74) Representative: Mahé, Jean et al
Postfach 3255 Grenzacherstrasse 124
CH-4002 Basel (CH)

(54) **Pyrrolylbenzodiazepinones and medicines containing them.**

(57) The present invention relates to compounds of the formula

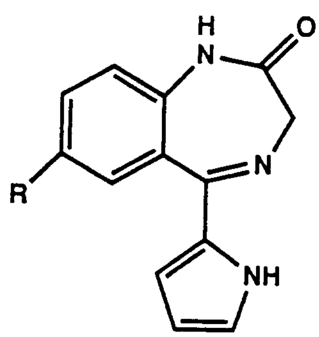

I

wherein R is methyl, fluorine or bromine,
and pharmaceutically acceptable salts thereof. Utilizable as therapeutically active agents especially for the treatment of retroviral infections, particularly HIV infections, they can be prepared in a manner known per se by a process which comprises reacting 2-bromo-2'-(1H-pyrrol-2-yl-carbonyl)acetanilide, correspondingly substituted by R in the 4'-position, with ammonia and cyclizing the obtained 2-aminoacetanilide with pivalic acid.

EP 0 552 665 A1

The present invention relates to a compound of the formula

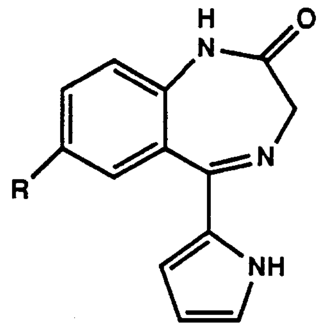

I

wherein R is methyl, fluorine or bromine,
and its pharmaceutically acceptable salts.

Objects of the invention are the above compounds per se and for use as therapeutically active agents especially for the treatment of retroviral infections, particularly HIV infections;

further a process for the manufacture of these compounds and medicaments containing one of such compounds, and

the use of these compounds for the manufacture of a medicament for the treatment of retroviral infections, particularly HIV infections.

The above compounds can be prepared in a manner known per se and as described in detail in Examples 1-3, by a process which comprises reacting a 4'-substituted 2-bromo-2'-(1H-pyrrol-2-ylcarbonyl)-acetanilide of formula

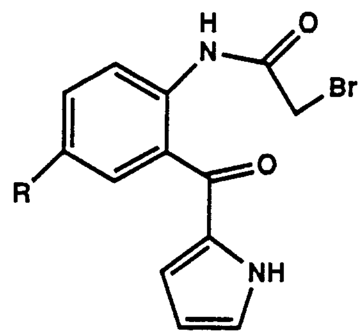

II

with ammonia and cyclizing the obtained 2-aminoacetanilide, e.g. with pivalic acid.

Examples of compounds of formula II are the compounds (4) and (11) in Figures 1 and 2. According to the above process a solution of a compound II, e.g. in THF and $CH_2Cl_2$, is reacted with ammonia. The obtained compound, an example of which is that of formula (5) in Fig. 1, is reacted with pivalic acid in a solvent, such as toluene or n-butanol, at a temperature up to the reflux temperature.

The preparation of the compounds II from known starting materials is shown in Fig. 1 and 2 and described in detail in Examples 1-3.

The compounds I and their salts have useful antiviral, especially anti-retroviral activity, particularly against HIV, the virus implicated in the development of AIDS and related diseases such as ARC (AIDS related complex). These compounds also inhibit HIV replication by inhibiting such important HIV viral functions as TAT (transactivating transcriptional) activity.

The antiviral activity of the compounds I can be shown as follows:

A plaque reduction assay was performed according to the procedures described by B.A. Larder, B. Chesebro and D.D. Richman in Antimicro. Agents and Chemother. 34, (1990), 436-441. A LAV-1 strain of HIV-1 was grown in CD4+ CEM cells. Monolayers of CD4+ HeLa cells were infected with the virus. After one hour absorption at 37°C, the compound being tested for antiviral activity was added in a culture medium with 5% fetal bovine serum and 0.1% DMSO, and the cultures were incubated at 37°C for 3 days. The cell monolayers were fixed with 10% formaldehyde and stained with 0.25% crystal violet to visualize plaques. Duplicate wells were prepared for each drug dilution and percent plaque reduction was calculated based on the control value without addition of drug.

The compounds tested were the compounds of formula I. Also tested for comparative purposes was the compound of formula I wherein R is chlorine (disclosed in U.S. Patent No. 5,041,438) and the RT inhibitor, AZT. Results of the assay are provided in Table 1 below:

Table 1

| Compound | Concentration (µM) | Average Number of Plaques Remaining After Treatment | % Reduction |
|---|---|---|---|
| Formula I wherein R = methyl | 10 | 4 | 95 |
| | 3.16 | 7 | 90 |
| | 1.0 | 12 | 84 |
| | 0.316 | 24 | 68 |
| | 0.1 | 55 | 27 |
| | 0.0316 | 79 | 0 |
| Formula I wherein R = bromine | 10 | 12 | 84 |
| | 3.16 | 34 | 55 |
| | 1.0 | 42 | 45 |
| | 0.316 | 69 | 9 |
| | 0.1 | 81 | 0 |
| Formula I wherein R = fluorine | 10 | 0 | 100 |
| | 3.16 | 11 | 85 |
| | 1.0 | 26 | 67 |
| | 0.316 | 35 | 54 |
| | 0.1 | 62 | 18 |
| | 0.00316 | 65 | 14 |
| Formula I wherein R = chlorine | 10 | 5 | 93 |
| | 3.16 | 9 | 88 |
| | 1.0 | 15 | 80 |
| | 0.316 | 33 | 57 |
| | 0.1 | 59 | 23 |
| | 0.00316 | 62 | 19 |
| AZT | 1.0 | 8 | 90 |
| | 0.1 | 22 | 71 |
| | 0.01 | 53 | 30 |
| | 0.001 | 76 | 0 |

An anti-virally effective amount of a compound of formula I for treating a retroviral infection is in the range of 0.1 to 10 mg/kg body weight per day. This dosage may be administered in one or more doses at various intervals such as 2, 4, 6, 8, 12, or 24 hours. The suitable dosage is one that achieves a therapeutic blood level of 0.05-10 µM, with 0.1 to 5 µM being preferred. This blood level may be best achieved by administering approximately 1-3 mg/kg body weight once or twice per day.

The compounds of formula I may be administered in various dosage forms as set forth herein. Either the compounds, compositions, or their pharmaceutically acceptable salts are suitable. Pharmaceutically acceptable salts may be salts of organic acids such as lactic, acetic, maleic, or p-toluenesulfonic acid and the like, as well as salts of pharmaceutically acceptable mineral acids such as hydrochloric and sulfuric acids and the like.

The compounds may also be administered with other anti-retroviral agents and particularly with known reverse transcriptase (RT) inhibitors such as ddC (2',3'-dideoxycytidine), AZT (3'-azido-3'-deoxythymidine), ddI (2',3'-dideoxyinosine), ddA (2',3'-dideoxyadenosine), or other inhibitors which act against other HIV proteins (e.g., protease, integrase and REV protein). The dosages of ddC and AZT used in AIDS or ARC patients have been published. A virustatic range of ddC is generally between 0.05 µM to 1.0 µM. A dosage range of about .005-0.25 mg/kg body weight is virustatic in most patients. The preliminary dose ranges for oral administration are somewhat broader, for example .001 to 0.25 mg/kg given in one or more doses at intervals of 2, 4, 6, 8, 12, etc. hours. Currently 0.01 mg/kg body weight ddC given every 8 hours is preferred. When given in combined therapy, the anti-RT compound may be given at the same time as a

compound of formula I or the dosing may be staggered as desired. The two drugs may also be combined in a composition. Doses of each may be less when used in combination than when they are used as a single agent.

The instant invention is also directed to compositions containing a therapeutically effective amount of a compound of formula I in a pharmaceutically acceptable carrier. It is possible for the compounds of the invention to be administered alone in solution. However, it is preferred that the active ingredients be administered in a pharmaceutical formulation. These formulations comprise at least one active ingredient together with one or more pharmaceutically acceptable carriers and/or other therapeutic agents, for example an RT inhibitor. These carriers include those well known in the art as suitable for oral, rectal, nasal, topical, buccal, sublingual, vaginal, or parenteral (including subcutaneous, intramuscular intravenous, and intradermal) administration. The compositions of the invention suitable for oral administration may consist of liquid solutions such as an effective amount of the compound dissolved in diluents such as water, saline, or orange juice.

The compositions may be conveniently presented in unit dosage forms which preferably comprise from 6-600 mg of a compound of formula I, most preferably from 60-180 mg.

Unit dosage forms for oral administration, such as capsules, sachets or tablets, may contain a predetermined amount of the active ingredient as a solid or granules, as a solution or suspension in an aqueous liquid, or in an oil-in-water emulsion or a water-in-oil liquid emulsion contained in, for example, soft gelatin capsules. Tablet unit dosage forms may include one or more of lactose, microcrystalline cellulose, colloidal silicon dioxide, croscarmellose sodium, magnesium stearate, stearic acid and other excipients, colorants, and pharmacologically compatible carriers. Unit dosage forms suitable for oral administration also include lozenges comprising the active ingredient in a flavor, usually sucrose and acacia or tragacanth; and pastilles comprising the active ingredient in an inert base such as gelatin and glycerin, or sucrose and acacia.

Unit dosage forms for rectal administration may be presented as a suppository with a suitable base comprising, for example, cocoa butter or a salicylate. Mouthwashes comprising the active ingredient in a suitable liquid carrier are also contemplated as a means of administration. Formulations suitable for vaginal administration may be presented as pessaries, tampons, creams, gels, pastes, foams, or spray formulas containing in addition to the active ingredient such carriers as are known in the art to be appropriate.

Formulations suitable for parenteral administration include aqueous and non-aqueous, isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampules and vials, and may be stored in a freeze-dried (lyophilized) condition requiring only the addition of the sterile liquid carrier, for example, water for injection, immediately prior to use. Extemporaneous injection solutions and suspensions may be prepared from sterile powders, granules and tablets of the kind previously described.

An anti-virally effective amount of the compounds or compositions of the invention can also be administered for alleviating the cytopathic destructive effects in a patient infected with a retrovirus. Since the compounds of formula I inhibit TAT, they can inhibit viral replication at the latent stage. The dosages mentioned previously would be suitable for this purpose. Preferably 1-3 mg/kg body weight given once or twice a day provides the virustatic range of 0.01-5.0 $\mu$M. The compounds can be administered to AIDS patients, ARC patients or asymptomatic HIV-infected patients.

Figure 1

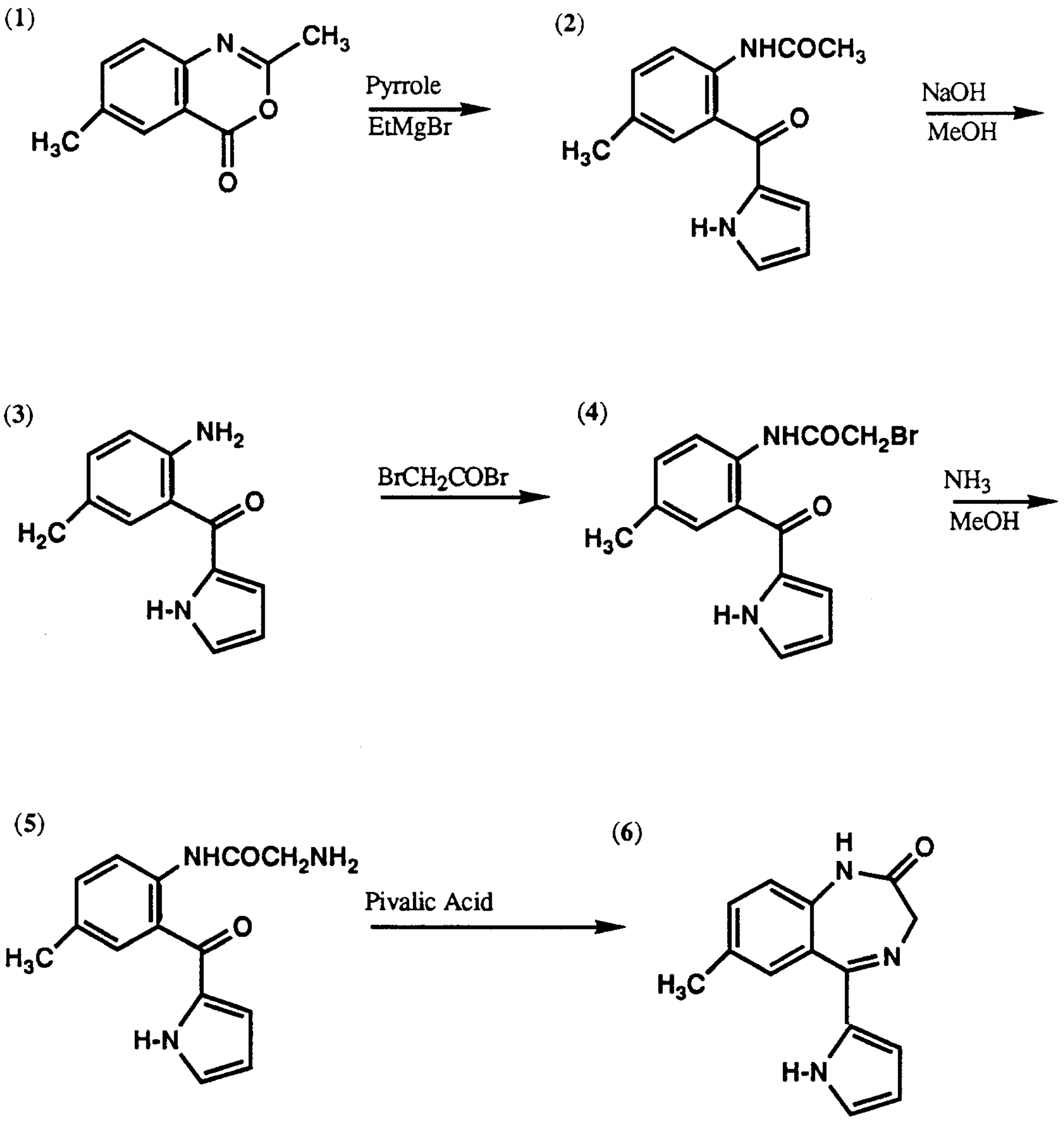


(1) (2) (3) (4) (5) (6)

Figure 2

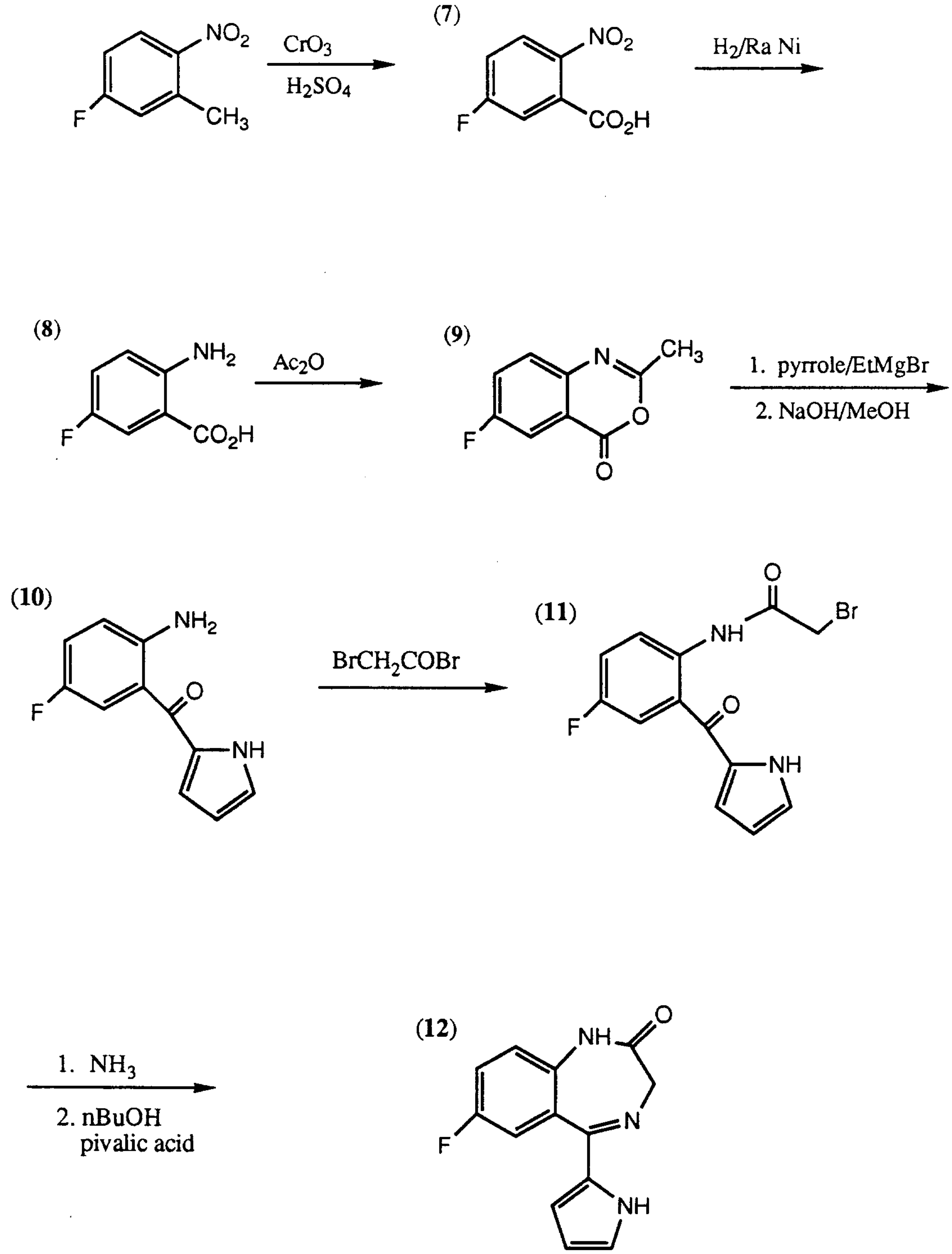


Example 1

The product of the present example may be prepared in accordance with the reaction scheme of Figure 1.

1a) A solution of 42.7 ml of 2.0M ethylmagnesium bromide in 130 ml of THF was cooled to -5 ° C and treated dropwise with a solution of 5.93g pyrrole in 20 ml of THF while maintaining the temperature at -5 ° C. After stirring at 0 ° C for 1 hr, a solution of 7.89g 2-methyl-6-methyl-4H-3,1-benzoxazin-4-one (**1**) in 75 ml of THF was added keeping the mixture below 15 ° C. The reaction was then heated to 60 ° C,

stirred at 60°C for 1 hr and at 50°C for 1 hr., and then cooled to room temperature. The reaction mixture was washed with saturated aqueous ammonium chloride, and with saturated sodium chloride solution. The organic layer was dried, treated with charcoal, filtered and evaporated. The solids were slurried in hexane, filtered and washed to give 8.092 g (74.1%) of (**2**).

1b) The solids were combined with 15 ml of 30% sodium hydroxide, 20 ml $H_2O$, and 40 ml methanol. The reaction was heated to reflux temperature for 2.5 hours. An additional 15 ml of 30% sodium hydroxide and 40 ml of MeOH was added and the mixture heated 2 hrs. After cooling to room temperature, 50 ml $H_20$ was added, and the mixture was stirred for 15 minutes. The solids were filtered and washed with $H_2O$ and hexane to give 5 g (75.1%) of (2-amino-5-methylphenyl)-(1H-pyrrol-2-yl)-methanone (**3**).

1c) To a stirred solution of 5.0 g of (**3**) in 100 ml of $CH_2Cl_2$ containing 9.1 g of sodium bicarbonate was added dropwise 3.16 ml of bromacetylbromide. After stirring at room temperature for 5 hrs., 3 ml bromoacetylbromide was added and the mixture stirred another 2 hrs. $H_2O$ was then added and the organic layer separated and washed with $H_2O$. The aqueous layers were combined and extracted with $CH_2Cl_2$. The combined organic layers were dried, filtered, and evaporated. The residue was dissolved in THF, treated with charcoal and then evaporated to 10 ml.

1d) This solution was diluted with 60 ml THF and added to 50 ml of liquid ammonia in a dry ice bath. The reaction mixture was stirred overnight and the ammonia allowed to evaporate. The remaining solvent was evaporated, and the residue was purified via flash column chromotography using $CH_2Cl_2$/MeOH (10:1) and $CH_2Cl_2$/MeOH (8:1) as eluant. The product was further purified by flash column chromatography using $CH_2Cl_2$/MeOH (6:1) as eluant to give 1.717 g (26.8%) of 2-amino-4'-methyl-2'(1H-pyrrol-2-yl carbonyl)-acetanilide (**5**).

1e) 1.717 g of (**5**) was combined with 50 ml toluene and 681 mg pivalic acid and heated to reflux temperature for 1 hr. The reaction was cooled to room temperature and combined with 50 ml THF and 20 ml ether and washed with saturated sodium bicarbonate solution. The organic layers were dried, filtered and evaporated. The residue was purified by flash column chromatography using EtOAc/petroleum ether (1:1) then EtOAc/petroleum ether (3:2) as eluant to give 240 mg of (**6**). This material was further purified by crystallization from EtOH to yield 162 mg (15.0%) of 1,3-dihydro-7-methyl-5-(1H-pyrrol-2-yl)-2H-1,4-benzodiazepin-2-one (**6**): mp = 237 - 239°C.

Example 2

The product of the present example may be prepared in accordance with the reaction scheme shown in Figure 2.

2a) To a stirred solution of 3.1 g of 5-fluoro-2-nitrotoluene in 20 ml of concentrated sulfuric acid was added 6.4 g of chromium trioxide in portions, while maintaining the reaction temperature at 35-40°C. The mixture was stirred for 1.5 h., then poured into stirred ice water, and extracted with ethyl acetate. The combined organic fractions were extracted with saturated aqueous sodium bicarbonate. The pH of the aqueous fraction was adjusted to 2 using concentrated phosphoric acid and the acidic mixture extracted with ethyl acetate. The organic layer was dried and evaporated to give 1.3g (50%) of (**7**).

2b) A solution of 1.6 g of 5-fluoro-2-nitrobenzoic acid (**7**) in 150 ml of methanol was hydrogenated under atmospheric pressure for two hours using Raney nickel as catalyst. The catalyst was removed by filtration and the solvent evaporated. The residue was purified by filtration over silica gel using 10% methanol-methylene chloride as eluant to give 0.8 g (60%) of (**8**).

2c) A mixture of 0.8 g of compound (**8**) in 10 ml of acetic anhydride was heated to reflux temperature overnight. The solvent was evaporated, and the residue purified by flash chromatography, using 25% ethyl acetate-75% hexane as eluant, to give 0.73 g (79%) of 2-methyl-6-fluoro-4H-3,1-benzoxazin-4-one (**9**).

2d) In a manner analogous to that of 1a) and 1b) above, from 0.7 ml (10.18 mmole) of pyrrole and 0.73 g of 2-methyl-6-fluoro-4H-3,1-benzoxazin-4-one (**9**), there were obtained 0.72 g (86%) of (2-amino-5-fluorophenyl)-1H-pyrrol-2-ylmethanone (**10**): mp = 90-92°C after filtration over silica gel using ethyl acetate-hexane (1:1) as eluant.

2e) In a manner analogous to that of 1c) above, from 0.42 g of (**10**) and 270 μL of bromoacetylbromide, there were obtained 650 mg (99%) of 2-bromo-4'-fluoro-2'-(1H-pyrrol-2-ylcarbonyl)-acetanilide (**11**): mp = 150-152°C.

2f) To 50 ml of condensed liquid ammonia in a dry ice-acetone bath was added a solution of 0.65g of compound (**11**) in 15 ml of methylene chloride and 3 ml of tetrahydrofuran. The reaction mixture was stirred overnight and the ammonia allowed to gradually evaporate. The dried residue was partitioned

between water and ethyl acetate. The layers were separated and the aqueous layer was extracted with ethyl acetate. The combined organic layers were washed with brine, dried and evaporated. The residue was combined with 30 ml of n-butanol and 50 mg of pivalic acid and heated to reflux temperature for 16 hours. The solvent was evaporated to dryness and the residue was crystallized from 10% methanol-90% methylene chloride to give 0.33 g (68%) of 7-fluoro-1,3-dihydro-5-(1H-pyrrol-2-yl)-2H-1,4 benzodiazepin-2-one (**12**): mp = 255-257 °C after treatment with charcoal in hot methanol.

Example 3

The corresponding 7-bromo compound may be obtained by using 5-bromo-2-nitrotoluene as the starting material.

The following galenical compositions can be prepared in a manner known per se:

Example 4

Formulations for compounds for Formula I

a)

| Tablet formulation I | |
|---|---|
| Ingredients | mg/Tablet |
| Active ingredient | 20 mg |
| Starch | 40 mg |
| Avicel | 80 mg |
| Lactose | 274 mg |
| Magnesium Stearate | 2 mg |
| | 416 mg |

b)

| Tablet formulation II | |
|---|---|
| Ingredients | mg/Tablet |
| Active Ingredient | 20 mg |
| Lactose | 180 mg |
| Pregelatinized Starch | 15 mg |

c)

| Soft gelatin capsule formulation | |
|---|---|
| Ingredients | mg/Capsule |
| Active ingredient | 20 mg |
| Ethoxylated Fatty acids | 500 mg |
| PEG 4000 | 100 mg |
| Vegetable Oils q.s. to | 1.0 g |

d)

| Oral liquid formulation | |
|---|---|
| Ingredients | mg/formulation |
| Active ingredient | 20.0 mg |
| Methylparaben | 20.0 mg |
| Sucrose | q.s. |
| Flavoring Agent | q.s. |
| Citrate Buffer | q.s. |
| Purified Water q.s. | 5.0 ml |

Example 5

Formulations for compounds of formula I and ddC

a)

| Tablet formulation I | |
|---|---|
| Ingredients | mg/Tablet |
| Formula I | 20 mg |
| ddC | 5 mg |
| Starch | 40 mg |
| Avicel | 80 mg |
| Lactose | 269 mg |
| Magnesium Stearate | 2 mg |
| | 416 mg |

b)

| Tablet formulation II | |
|---|---|
| Ingredients | mg/Tablet |
| Formula I | 20 mg |
| ddC | 5 mg |
| Lactose | 175 mg |
| Pregelatinized Starch | 15 mg |
| Microcrystalline Cellulose | 72 mg |
| Modified Starch | 10 mg |
| Magnesium Stearate | 3 mg |
| | 300 mg |

c)

| Soft gelatin capsule formulation | |
|---|---|
| Ingredients | mg/capsule |
| Formula I | 20 mg |
| ddC | 5 mg |
| Ethoxylated Fatty acids | 500 mg |
| PEG 4000 | 100 mg |
| Vegetable Oils q.s. to | 1.0 g |

d)

| Oral liquid formulation | |
|---|---|
| Ingredients | mg/formulation |
| Formula I | 4.0 mg |
| ddC | 1.0 mg |
| Methylparaben | 2.0 mg |
| Propylparaben | 0.2 mg |
| Sucrose | 100.0 q.s. |
| Flavoring Agent | q.s. |
| Citrate Buffer | 5.0 mg |
| Purified Water q.s. | 1.0 ml |

e)

| Parenteral formulation | |
|---|---|
| Ingredients | mg/formulation |
| Formula I | 20.0 mg |
| ddC | 5.0 mg |
| Propylene Glycol | 20.0 mg |
| Emulphor | 2.0 mg |
| Water for Injection q.s | 1.0 mg |

## Claims

1. A compound of the formula

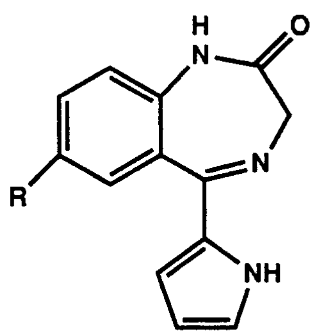

I

wherein R is methyl, fluorine or bromine,
and its pharmaceutically acceptable salts.

2. A compound as in claim 1 of the group of the following:
1,3-dihydro-7-methyl-5-(1H-pyrrol-2-yl)-2H-1,4-benzodiazepin-2-one,
7-fluoro-1,3-dihydro-5-(1H-pyyrol-2-yl)-2H-1,4-benzodiazepin-2-one,
7-bromo-1,3-dihydro-5-(1H-pyrrol-2-yl)-2H-1,4-benzodiazepin-2-one.

3. A compound as in claim 1 or 2 for use as a therapeutically active agent, especially for the treatment of retroviral infections, particularly HIV infections.

4. A process for preparing a compound of formula I as in claim 1, which comprises reacting a 4'-substituted 2-bromo-2'-(1H-pyrrol-2-yl-carbonyl)acetanilide of formula

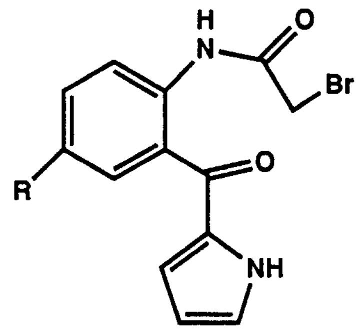

II

with ammonia and cyclizing the obtained 2-aminoacetanilide.

5. A medicament, especially for the treatment of retroviral infections, particularly HIV infections, containing as active ingredient a compound as in claim 1 or 2.

6. The use of a compound as in claim 1 or 2, for the manufacture of a medicament for the treatment of retroviral infections, particularly HIV infections.

European Patent Office

# EUROPEAN SEARCH REPORT

Application Number

EP 93 10 0451

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | EP-A-0 429 868 (HOFFMAN-LA ROCHE)<br>* the whole document * | 1-3 | C07D403/04<br>A61K31/55 |
| A | DE-A-2 017 060 (SUMITOMO)<br>* page 33; claims * | 1-4 | |
| P,X | EP-A-0 491 218 (HOFFMAN-LA ROCHE)<br>* the whole document * | 1-4 | |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 22 APRIL 1993 | FRANCOIS J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)